# EUROPEAN PATENT SPECIFICATION

(11) **EP 4 463 465 B1**
(45) Date of publication and mention of the grant of the patent: **07.01.2026**
(21) Application number: 23700764.6
(22) Date of filing: 12.01.2023
(51) Int. Cl.: C07K 14/00, C07K 1/22

(54) **HIGH AFFINITY PURIFICATION OF FERRITIN**
AFFINITÄTSREINIGUNG VON FERRITIN
PURIFICATION D'AFFINITÉ DE FERRITINE

(30) Priority: 12.01.2022 EP 22151109
(43) Date of publication of application: 20.11.2024
(73) Proprietor: NAVIGO PROTEINS GMBH, 06120 Halle/Saale (DE)
(72) Inventor: BOECKER, Heike, 06120 Halle/Saale (DE); COBURGER, Ina, 06120 Halle/Saale (DE); BOBOLOWSKI, Hanna, 06120 Halle/Saale (DE); LOTZE, Jonathan, 06120 Halle/Saale (DE)
(86) International application number: PCT/EP2023/050586
(87) International publication number: WO 2023/135187

(56) References cited:
- WO-A1-2021/122943
- WO-A2-2013/055058
- CONLON-HOLLINGSHEAD C ET AL: "Rapid one-step purification of ferritin from cell-free translation systems", ANALYTICAL BIOCHEMISTRY, ACADEMIC PRESS, AMSTERDAM, NL, vol. 120, no. 2, 1 March 1982 (1982-03-01), pages 235 - 242, XP024818239, ISSN: 0003-2697, [retrieved on 19820301], DOI: 10.1016/0003-2697(82)90341-4
- KOICHI ORINO ET AL: "Purification and Characterization of Canine Serum Ferritin-binding Proteins", BIOMETALS, KLUWER ACADEMIC PUBLISHERS, BO, vol. 19, no. 3, 1 June 2006 (2006-06-01), pages 315 - 322, XP019391829, ISSN: 1572-8773, DOI: 10.1007/S10534-005-1299-Z

## Description

### TECHNICAL FIELD

The present invention relates to novel proteins that specifically bind to the ferritin. The novel proteins of the present invention are advanced and powerful tools because they allow precise purification methods of ferritin, for example via affinity chromatography. Further, the binding protein for ferritin is useful for methods to analyze the presence of ferritin.

### BACKGROUND OF THE INVENTION

Ferritin, a large multi-subunit protein complex, is the main intracellular storage protein for iron. Iron is an essential nutrient required for oxygen carriage in the bloodstream and as component of enzymes for oxidative respiration. Iron deficiencies have severe effects for example on the neurological development of children.

The iron storage and iron transport protein is mainly located in liver, spleen, and bone marrow but also in blood serum and plasma. The protein consists of subunits of four helix bundle proteins that arrange to a 24mer in strict symmetry.

Purified ferritin can be administered to individuals in need of iron and can be used to treat or supplement iron deficiency disorders.

Further, Ferritin was described as part of a drug delivery vehicle for tumor targeting therapy. WO 2021/122943 A1 relates to the field of protein purification and provides proteins that bind specifically to acid alpha glucosidase (GAA) and affinity matrices comprising the GAA binding proteins, respectively for affinity purification of GAA.

Conlon-Hollingshead et al., Rapid one-step purification of ferritin from cell free translation systems, Analytical Biochemistry, 1982, Vol. 120(2), pp. 235-242, discloses a method for purifying ferritin by anti-ferritin affinity column chromatography, based on ferritin antibodies.

Thus, there is a strong need in the art to provide methods for an efficient purification of ferritin. The present invention meets this need by providing novel binding proteins for ferritin that are particularly advantageous because they allow a precise purification of ferritin, in particular via affinity chromatography, for further uses in medical applications.

The above overview does not necessarily describe all problems solved by the present invention.

### SUMMARY OF THE INVENTION

This summary of the invention is not limiting, and other aspects and embodiments of the invention will become evident from the following description, examples and drawings.

The present invention is set out in the appended set of claims. In particular, the present invention provides a binding protein for ferritin comprising an amino acid sequence with at least 90 % sequence identity to SEQ ID NO: 2 or SEQ ID NO: 3, wherein the binding protein has a binding affinity of less than 100 nM for ferritin.

### BRIEF DESCRIPTION OF THE FIGURES

**FIGURE 1****:** Amino acid sequences of binding proteins for ferritin (SEQ ID NOs: 2, 3, and 4). Identical and similar amino acids are labeled by a star (*) and a double point (:), respectively. The alignment was done using a multiple sequence alignment tool.
**FIGURE 2****:** SPR of 219127 (fusion protein comprising SEQ ID NO: 2) immobilized via c-terminal cysteine residue using PDEA coupling. 219127 binds to ferritin with an affinity below 0.5 nM even at pH 4.5.

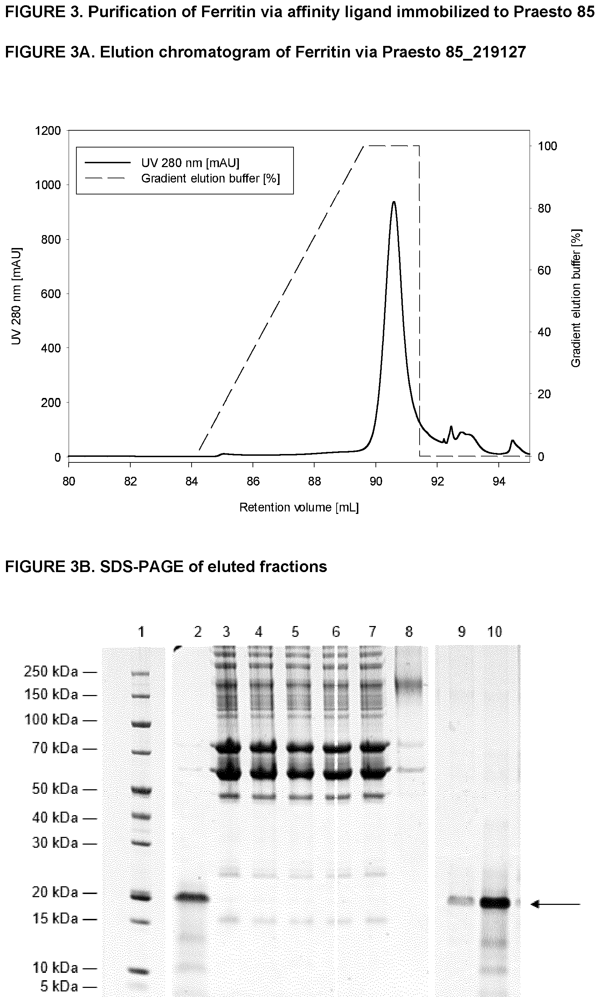

**FIGURE 3****:** Purification of ferritin via affinity ligand immobilized to Praesto 85. **FIGURE 3A** shows an elution chromatogram of an affinity chromatography run performed with fusion protein 219127 coupled to Praesto85 resin. The solid black line indicates UV 280 nm signal. The gradient of the elution buffer is shown as dashed black line. The figure shows a homogenous elution profile for ferritin with a singular peak. The peak maximum was at pH 3.1 in a pH gradient profile. **FIGURE 3B** shows an SDS-PAGE of analyzed fractions of an affinity chromatography run using Praesto85-219127. A liquid containing ferritin was loaded. Purified human ferritin is visible at MW band 20 kDa (arrow). No significant impurities were detectable. Lane 1: protein marker, lane 2: ferritin, lane 3-7: flowthrough fractions, lane 8: wash fractions (wash at pH 4.5), lane 9-10 elution fractions.

### DETAILED DESCRIPTION OF THE INVENTION

The present invention provides novel proteins having specific binding affinity for ferritin as defined in the appended claims. The novel proteins of the present invention are particularly advantageous because as affinity ligands for ferritin, they allow precise purification of ferritin, for example in affinity chromatography. Any polypeptide selected from the group of SEQ ID NO: 1, SEQ ID NO: 2, SEQ ID NO: 3, and SEQ ID NO: 4, or an amino acid sequence with at least 90 % identity to any one of SEQ ID NO: 1, SEQ ID NO: 2, SEQ ID NO: 3, and SEQ ID NO: 4, respectively, bind to human ferritin with high affinity.

Before the present invention is described in more detail below it is to be understood that this invention is not limited to the particular methodology, protocols and reagents described herein as these may vary. It is also to be understood that the terminology used herein is for the purpose of describing particular aspects and embodiments only and is not intended to limit the scope of the present invention, which is reflected by the appended items. Unless defined otherwise, all technical and scientific terms used herein have the same meaning as commonly understood by one of ordinary skill in the art to which this invention belongs. This includes a skilled person working in the field of protein engineering and purification, but also including a skilled person working in the field of developing new specific binding molecules for ferritin for use in technical applications, for example for use as affinity ligands in affinity chromatography.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Throughout this specification and the items, which follow, unless the context requires otherwise, the word "comprise", and variants such as "comprises" and "comprising", will be understood to imply the inclusion of a stated integer or step, or group of integers or steps, but not the exclusion of any other integer or step or group of integers or steps. The term "comprise(s)" or "comprising" may encompass a limitation to "consists of" or "consisting of", should such a limitation be necessary for any reason and to any extent.

Several documents (for example: patents, patent applications, scientific publications, manufacturer's specifications, instructions, UniProt Accession Number, etc.) may be cited throughout the present specification. Nothing herein is to be construed as an admission that the invention is not entitled to antedate such disclosure by virtue of prior invention.

All sequences referred to herein are disclosed in the attached sequence listing that, with its whole content and disclosure, forms part of the disclosure content of the present specification.

### General Definitions of Important terms used in the Application

The term "ferritin" refers to an amino acid sequence as shown in UniProtKB Q8TD27. The term "ferritin" comprises all polypeptides which show an amino acid sequence identity of at least 70 %, 80 %, 85 %, 90 %, 95 %, 96 % or 97 % or more, or 100 % to UniProtKB Q8TD27. In some embodiments, ferritin relates to mammalian ferritin. In some embodiments, ferritin relates to human ferritin.

The terms "binding protein for ferritin", "ferritin binding protein" or "affinity ligand"" may be used interchangeably herein and describe a protein that is capable to bind to ferritin. As described herein, a binding protein for ferritin refers to a protein with detectable interaction with ferritin, as determined by suitable methods such as for example SPR analysis or BLI or other appropriate technology known to someone skilled in the art.

The term "non-ferritin binding protein" refers to a protein with no detectable interaction with ferritin, as determined by suitable methods such as for example SPR analysis or BLI or other appropriate technology known to someone skilled in the art.

The term "binding affinity" refers to the ability of a polypeptide of the invention to bind to ferritin. Binding affinity is typically measured and reported by the equilibrium dissociation constant (K_{D}) which is used to evaluate and rank the strength of bimolecular interactions. The binding affinity and dissociation constants can be measured quantitatively. Methods for determining binding affinities are well known to the skilled person and can be selected, for instance, from the following methods that are well established in the art: surface plasmon resonance (SPR), Bio-layer interferometry (BLI), enzyme-linked immunosorbent assay (ELISA), kinetic exclusion analysis (KinExA assay), flow cytometry, fluorescence spectroscopy techniques, isothermal titration calorimetry (ITC), analytical ultracentrifugation, radioimmunoassay (RIA or IRMA), and enhanced chemiluminescence (ECL). Typically, the dissociation constant K_{D} is determined at temperatures in the range of 20°C and 30°C. If not specifically indicated otherwise, K_{D} values recited herein are determined at 25°C by SPR. In various embodiments of the present invention, the binding affinity for ferritin may be determined by the Sierra SPR-32 system (Bruker).

The term "fusion protein" relates to a protein comprising at least a first protein joined genetically to at least a second protein. A fusion protein is created through joining of two or more genes that originally coded for separate proteins. Thus, a fusion protein may comprise a multimer of identical or different proteins which are expressed as a single, linear polypeptide.

The term "amino acid sequence identity" refers to a quantitative comparison of the identity (or differences) of the amino acid sequences of two or more proteins. "Percent (%) amino acid sequence identity" with respect to a reference polypeptide sequence is defined as the percentage of amino acid residues in a sequence that are identical with the amino acid residues in the reference polypeptide sequence, after aligning the sequences and introducing gaps, if necessary, to achieve the maximum percent sequence identity. To determine the sequence identity, the sequence of a query protein is aligned to the sequence of a reference protein or polypeptide, for example, to the polypeptide of SEQ ID NO: 1. Methods for sequence alignment are well known in the art. For example, for determining the extent of an amino acid sequence identity of an arbitrary polypeptide relative to the amino acid sequence of, for example, SEQ ID NO: 1, the SIM Local similarity program is preferably employed (Xiaoquin Huang and Webb Miller (1991), Advances in Applied Mathematics, vol. 12: 337-357), that is freely available. For multiple alignment analysis, ClustalW is preferably used (Thompson et al. (1994) Nucleic Acids Res., 22(22): 4673-4680).

The terms "protein" and "polypeptide" refer to any chain of two or more amino acids linked by peptide bonds and does not refer to a specific length of the product. Thus, "peptides", "protein", "amino acid chain", or any other term used to refer to a chain of two or more amino acids, are included within the definition of "polypeptide", and the term "polypeptide" may be used instead of, or interchangeably with, any of these terms. The term "polypeptide" is also intended to refer to the products of post-translational modifications of the polypeptide like, e.g., glycosylation, which are well known in the art.

The term "alkaline stability" refers to the ability of the binding protein for ferritin to withstand alkaline conditions without significantly losing the ability to bind ferritin. The skilled person in this field can easily test alkaline stability by incubating a binding protein for ferritin with sodium hydroxide solutions, e.g., as described in the Examples, and subsequent testing of the binding affinity to ferritin by routine experiments known to someone skilled in the art, for example, by chromatographic approaches.

The term "chromatography" refers to separation technologies which employ a mobile phase and a stationary phase to separate one type of molecules (e.g., ferritin) from other molecules in the sample. The liquid mobile phase contains a mixture of molecules and transports these across or through a stationary phase (such as a solid matrix). Due to the differential interaction of the different molecules in the mobile phase with the stationary phase, molecules in the mobile phase can be separated.

The term "affinity chromatography" refers to a specific mode of chromatography in which a ligand (i.e. a binding protein for ferritin) coupled to a stationary phase interacts with a molecule (i.e. ferritin) in the mobile phase (the liquid sample), i.e. the ligand has a specific binding affinity for the molecule to be captured. As understood in the context of the invention, affinity chromatography involves the addition of a (liquid) sample containing ferritin to a stationary phase which comprises a chromatography ligand, such as a binding protein for ferritin. The terms "solid support" or "solid matrix" are used interchangeably for the stationary phase.

The terms "affinity matrix" or "affinity purification matrix", as used interchangeably herein, refer to a matrix, e.g., a chromatographic matrix, onto which an affinity ligand (e.g., a binding protein for ferritin or a fusion protein comprising a binding protein for ferritin) is attached. The attached affinity ligand is capable of specific binding to a molecule of interest (e.g., ferritin) which is to be purified or removed from a liquid. The liquid may, for example but not limited to, be blood serum or plasma. The term "affinity purification" as used herein refers to a method of purifying (capturing) ferritin from a liquid by binding ferritin to a ligand for ferritin that is immobilized to a matrix. Thereby, ferritin is removed from the liquid.

### Detailed description of the embodiments of the invention

The present invention will now be further described. In the following passages different aspects of the invention are defined in more detail. Each aspect defined below may be combined with any other aspect or aspects unless clearly indicated to the contrary. In particular, any feature indicated as being preferred or advantageous may be combined with any other feature or features indicated as being preferred or advantageous.

The novel binding protein for ferritin exhibits a specific binding affinity for the ferritin. The binding protein for ferritin according to the invention comprises the amino acid sequence of SEQ ID NO: 2 or SEQ ID NO: 3, or an amino acid with at least 90 % sequence identity to SEQ ID NO: 2 or SEQ ID NO: 3.

Further disclosed herein, but not forming part of the invention, is a binding protein for ferritin comprising the amino acid sequence of SEQ ID NO: 1, or an amino acid with at least 90 % sequence identity to SEQ ID NO: 1.

In some embodiments of the present invention, the binding protein for ferritin comprises the amino acid sequence of SEQ ID NO: 2. In some embodiments, the binding protein for ferritin comprises an amino acid with at least 90 % sequence identity to SEQ ID NO: 2.

In some embodiments of the present invention, the binding protein for ferritin comprises the amino acid sequence of SEQ ID NO: 3, or an amino acid with at least 90 % sequence identity to SEQ ID NO: 3.

Also disclosed herein, but not forming part of the invention, is a binding protein for ferritin that comprises the amino acid sequence of SEQ ID NO: 4, or an amino acid with at least 90 % sequence identity to SEQ ID NO: 4.

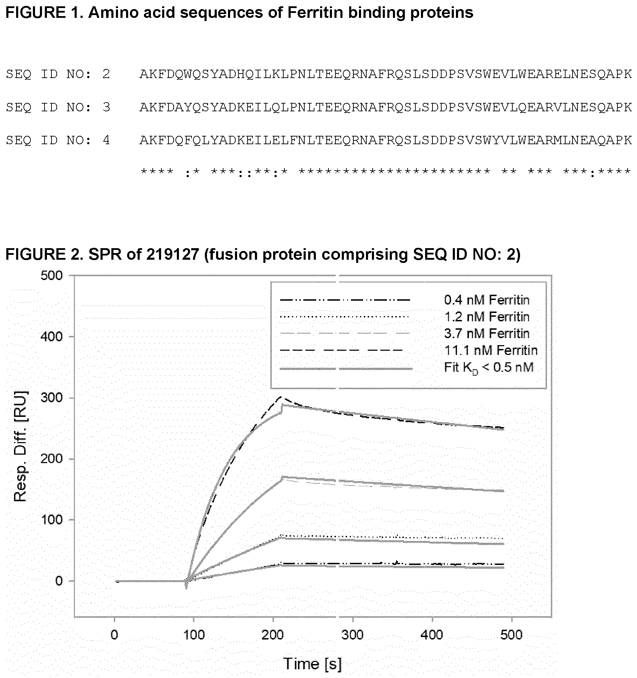

A binding protein for ferritin as disclosed herein comprises at least one amino acid sequence as shown in **FIGURE 1****.**

The binding protein for ferritin according to the invention has at least 90 %, 91 %, 92 %, 93 %, 94 %, 95%, 96 %, 97 %, 98 %, 99 %, or 100 % sequence identity to the amino sequence of SEQ ID NOs: 2 or 3.

Amino acids corresponding to positions 1-4, 7, 9-11, 14, 15, 17, 19-40, 42, 43, 45-47, 49-51, and 53-56 are identical in ferritin binding proteins as shown in the amino acid sequences of SEQ ID NOs: 1-4.

The amino acid corresponding to position 5 of SEQ ID NO: 1 may be selected from Q or A.

The amino acid corresponding to position 6 of SEQ ID NO: 1 may be selected from an aromatic amino acid (W, Y, F).

The amino acid corresponding to position 8 of SEQ ID NO: 1 may be selected from S or L, preferably S.

The amino acid corresponding to position 12 of SEQ ID NO: 1 may be selected from K or H.

The amino acid corresponding to position 13 of SEQ ID NO: 1 may be selected from E or Q. The amino acid corresponding to position 16 of SEQ ID NO: 1 may be selected from K, E, or Q. The amino acid corresponding to position 18 of SEQ ID NO: 1 may be selected from P or F, preferably P.

The amino acid corresponding to position 41 of SEQ ID NO: 1 may be selected from E or Y, preferably E.

The amino acid corresponding to position 44 of SEQ ID NO: 1 may be selected from W or Q. The amino acid corresponding to position 48 of SEQ ID NO: 1 may be selected from E, V, or M. The amino acid corresponding to position 52 of SEQ ID NO: 1 may be selected from S or A, preferably S.

One embodiment refers to a binding protein for ferritin comprising an amino acid sequence with at least 90 % sequence identity to SEQ ID NO: 2 or SEQ ID NO: 3.

One embodiment refers to a binding protein for ferritin comprising an amino acid sequence with at least 90 % sequence identity to SEQ ID NO: 2.

In some embodiments, the binding protein for ferritin has at least 90 %, 91 %, 92 %, 93 %, 94 %, 95%, 96 %, 97 %, 98 %, 99 %, or 100 % sequence identity to SEQ ID NO: 2 or SEQ ID NO: 3 wherein amino acids corresponding to positions 1-4, 7-11, 14, 15, 17- 43, 45-47, 49-56 of SEQ ID NO: 2 or SEQ ID NO: 3 are not modified.

In some embodiments, the binding protein for ferritin has at least 90 %, 91 %, 92 %, 93 %, 94 %, 95%, 96 %, 97 %, 98 %, 99 %, or 100 % sequence identity to SEQ ID NO: 2 or SEQ ID NO: 3 wherein one or more amino acids corresponding to positions 5, 6, 8, 12, 13, 16, 44, 48 of SEQ ID NO: 2 or SEQ ID NO: 3 are modified.

One advantage of the herein disclosed binding protein for ferritin is the important functional characteristic that it binds specifically to ferritin at low affinity. Needless to point out, that this is of particular advantage in purifying ferritin in the process of protein purification. The binding protein for ferritin is functionally characterized by a binding affinity of less than 100 nM for ferritin. In some embodiments, the binding protein for ferritin is functionally characterized by a binding affinity of less than 50 nM for human ferritin. In some embodiments, the binding protein for ferritin is functionally characterized by a binding affinity of less than 10 nM for human ferritin.

**Multimers.** In one embodiment of the invention, the binding protein for ferritin comprises 1, 2, 3, 4, 5, or 6 binding protein(s) linked to each other. In one embodiment of the invention, the binding protein for ferritin comprises one ferritin binding protein or two ferritin binding proteins linked to each other. Multimers of the binding protein are generated artificially, generally by recombinant DNA technology well-known to a skilled person. In some embodiments, the multimer is a homo-multimer, e.g. the amino acid sequences of binding protein for ferritin are identical. In other embodiments, the multimer is a hetero-multimer, e.g. the amino acid sequences of the binding protein for ferritin are different.

**Fusion proteins.** In some embodiments, the binding protein for ferritin as described above is fused to one further polypeptide distinct from the polypeptide as disclosed. In various embodiments, the further polypeptide distinct from the binding protein for ferritin as disclosed herein might be a non-ferritin binding protein. In some embodiments, the further non-ferritin binding polypeptide is a non-Immunoglobulin (Ig)-binding protein, for example but not limited to, a protein that does not bind to Ig. In some embodiments, a common structural feature of the non-ferritin binding proteins is that they have a triple helical Protein A-like structure comparable to the structure of the ferritin binding proteins as disclosed herein.

In some embodiments, a non-ferritin binding protein has an amino acid sequence identity of at least 80 %, 81 %, 82 %, 83 %, 84 %, 85 %, 86 %, 87 %, 88 %, 89 %, 90 %, 91 %, 92 %, 93 %, 94 %, 95 %, 96 %, 97 %, 98 %, 99 %, or 100 % to SEQ ID NO: 11. In some embodiments, a non-ferritin binding protein has an amino acid sequence identity of at least 89 % to SEQ ID NO: 11. Some embodiments encompass fusion proteins comprising a binding protein for ferritin as disclosed herein and at least one non-ferritin binding polypeptide.

Some embodiments encompass fusion proteins comprising a binding protein for ferritin as disclosed herein and one or two or more non-ferritin binding polypeptide(s).

As described herein, fusion proteins may comprise at least one binding protein for ferritin of any one selected from SEQ ID NOs: 1-4 and at least one non-ferritin binding protein.

As described herein, fusion proteins may -comprise at least one binding protein for ferritin of any one selected from SEQ ID NOs: 1-4 and at least one non-ferritin binding protein of at least 89% identity to SEQ ID NO: 11.

In some embodiments, a fusion protein of the invention comprises a binding protein for ferritin of the invention, wherein the C-terminus of the binding protein for ferritin is fused to the N-terminus of a non-ferritin binding protein. In some embodiments, a fusion protein of the invention comprises at least one binding protein for ferritin of the invention, wherein the C-terminus of the binding protein for ferritin is fused to the N-terminus of a dimer of a non-ferritin binding protein with at least 89 % amino acid sequence identity to SEQ ID NO: 11 or at least 90% amino acid sequence identity to SEQ ID NO: 11.

In one embodiment, the fusion protein comprises SEQ ID NO: 2 fused to two non-ferritin binding proteins (for example, see SEQ ID NO: 5 (219126)).

In one embodiment, the fusion protein comprises a dimer of SEQ ID NO: 2 fused to two non-ferritin binding proteins (for example, see SEQ ID NO: 6 (219127)).

In one embodiment, the fusion protein comprises SEQ ID NO: 3 fused to two non-ferritin binding proteins (for example, see SEQ ID NO: 7 (219124)).

In one embodiment, the fusion protein comprises a dimer of SEQ ID NO: 3 fused to two non-ferritin binding proteins (for example, see SEQ ID NO: 8 (219125)).

A fusion protein as further disclosed herein may comprise SEQ ID NO: 4 fused to two non-ferritin binding proteins (for example, see SEQ ID NO: 9 (219122)).

A fusion protein as further disclosed herein may comprise a dimer of SEQ ID NO: 4 fused to two non-ferritin binding proteins (for example, see SEQ ID NO: 10 (219123)).

In some embodiments said fusion protein comprises an attachment site for site-specific coupling to a solid support, as further described below.

**Molecules for purification or detection.** In some embodiments, the binding protein for ferritin may also comprise additional amino acid residues at the N- and/or C-terminal end, such as for example an additional sequence at the N- and/or C-terminal end. Additional sequences may include for example sequences introduced e.g. for purification or detection. Typical examples for such sequences include, without being limiting, Strep-tags, oligohistidine-tags, glutathione S-transferase, maltose-binding protein, inteins, intein fragments, or the albumin-binding domain of protein G, or others. In one embodiment, additional amino acid sequences include one or more peptide sequences that confer an affinity to certain chromatography column materials. The binding protein for ferritin or a fusion protein comprising the binding protein for ferritin may include specific attachment sites for the attachment to solid supports, preferably at the C-terminal end, such as cysteine or lysine.

**Use of the novel binding protein for ferritin in technical applications.** Also provided herein is the use of any novel binding protein for ferritin as disclosed herein, including fusion proteins, in technical applications, preferably for use in affinity purification.

**Affinity purification of ferritin.** As described herein, affinity chromatography (also called affinity purification) makes use of specific binding interactions between molecules. Methods for immobilization of protein and methods for affinity chromatography are well-known in the field of protein purification and can be easily performed by a skilled person in this field using standard techniques and equipment.

Some embodiments refer to a method of purification of ferritin, the method comprising: (i) providing a liquid that contains a ferritin; (ii) providing an affinity separation matrix comprising at least one binding protein for ferritin as described above or the fusion protein as described above coupled to said affinity separation matrix; (iii) contacting said affinity separation matrix with the liquid under conditions that permit binding of the at least one binding protein for ferritin as described above or the fusion protein as described above; and (iv) eluting said ferritin from said affinity purification matrix.

Some embodiments refer to a method of purification of ferritin, the method comprising: (i) providing a liquid that contains a ferritin wherein the liquid is blood plasma or blood serum or obtained from blood plasma or blood serum; (ii) providing an affinity separation matrix comprising at least one binding protein for ferritin as described above or the fusion protein as described above coupled to said affinity separation matrix; (iii) contacting said affinity separation matrix with the liquid under conditions that permit binding of the at least one binding protein for ferritin as described above or the fusion protein as described above; and (iv) eluting said ferritin from said affinity purification matrix.

In various embodiments, the method of affinity purification may further comprise one or more washing steps. Affinity purification matrices suitable for the disclosed uses and methods are known to a person skilled in the art.

In various embodiments, the method of affinity purification may further comprise one or more steps for isolating the purified ferritin protein via methods that are known to a person skilled in the art.

**Conjugation to a solid support.** In various aspects and/or embodiments of the present invention, the novel ferritin binding proteins disclosed herein including novel ferritin binding proteins generated or obtained by any of the methods as described above are conjugated to a solid support. In some embodiments, the ferritin binding protein comprises an attachment site for site-specific covalent coupling of the ferritin binding protein to a solid support. Specific attachment sites comprise without being limited thereto, natural amino acids, such as cysteine or lysine, which enable specific chemical reactions with a reactive group of the solid phase, or a linker between the solid phase and the protein.

**Affinity purification matrix.** In another embodiment, an affinity purification matrix is provided comprising a binding protein for ferritin, or a fusion protein comprising the binding protein for ferritin.

In preferred embodiments, the affinity purification matrix is a solid support. The affinity purification matrix comprises at least one binding protein for ferritin or a fusion protein comprising the binding protein for ferritin as described herein. Accordingly, a novel binding protein for ferritin disclosed herein or a fusion protein comprising the binding protein for ferritin is encompassed for use in the purification of ferritin by an affinity purification matrix.

Solid support matrices for affinity chromatography are known in the art and include, *e.g*., without being limited thereto, agarose and stabilized derivatives of agarose, cellulose or derivatives of cellulose, controlled pore glass, monolith, silica, zirconium oxide, titanium oxide, or synthetic polymers, and hydrogels of various compositions and combinations of the above.

The formats for solid support matrices can be of any suitable well-known kind. Such solid support matrix for coupling a novel protein or polypeptide of the present invention might comprise, *e.g*., one of the following, without being limited thereto: columns, capillaries, particles, membranes, filters, monoliths, fibers, pads, gels, slides, plates, cassettes, or any other format commonly used in chromatography and known to someone skilled in the art.

In one embodiment, the matrix is comprised of substantially spherical beads, for example Sepharose or Agarose beads. Matrices in particle form can be used as a packed bed or in a suspended form including expanded beds. In other embodiments of the invention, the solid support matrix is a membrane, for example a hydrogel membrane. In some embodiments, the affinity purification may involve a membrane as a matrix to which the binding protein for ferritin as described herein is covalently bound. The solid support can also be in the form of a membrane in a cartridge.

In some embodiments, the affinity purification involves a chromatography column containing a solid support matrix to which a novel protein of the present invention is covalently bound. A ferritin binding protein or a fusion protein comprising the ferritin binding protein as described above may be attached to a suitable solid support matrix via conventional coupling techniques. Methods for immobilization of protein ligands to solid supports are well-known in the field of protein engineering and purification and can easily be performed by a skilled person in this field using standard techniques and equipment.

**Use and *in vitro* methods to determine the presence of a ferritin.** In some embodiments refer to a use of the binding protein for ferritin as described above or the fusion protein as described above or the affinity matrix as described above in *in vitro* methods to determine the presence of ferritin.

Some embodiments, the binding protein for ferritin as described herein or the fusion protein as described herein is used in methods to determine the presence of a ferritin. Some embodiments relate to a method of analyzing the presence of ferritin in liquid samples, the method comprising the following steps: (i) providing a liquid that contains ferritin, (ii) providing the binding protein for ferritin, (iii) contacting the liquid that contains ferritin with the binding protein for ferritin or a fusion protein comprising the ferritin binding protein as described herein under conditions that permit binding of the at least one binding protein for ferritin to ferritin, (iv) isolating (eluting) the complex of a ferritin and the binding protein for ferritin or a fusion protein comprising the ferritin binding protein, and (v) determining the amount of the binding protein for ferritin which indicates the amount of ferritin in the liquid of (i).

**Method of quantification of a ferritin.** Further embodiments relate to a method of quantification of a ferritin, the method comprising: (i) providing a liquid that contains ferritin; (ii) providing a matrix to which the binding protein for ferritin or a fusion protein comprising the ferritin binding protein as described herein has been covalently coupled; (iii) contacting said affinity purification matrix with the liquid under conditions that permit binding of the at least one binding protein for ferritin or a fusion protein comprising at least one ferritin binding protein to ferritin; (iv) eluting said ferritin; and (v) quantitating the amount of eluted ferritin. Methods to determine the presence of ferritin in liquid samples might be quantitative or qualitative. Such methods are well known to the skilled person and can be selected, for instance but limited to, from the following methods that are well established in the art: enzyme-linked immunosorbent assay (ELISA), enzymatic reactions, surface plasmon resonance (SPR), or chromatography.

**Polynucleotides, vectors, host cells.** One embodiment covers an isolated polynucleotide or nucleic acid molecule encoding a binding protein for ferritin or a fusion protein comprising a binding for ferritin as described herein. A further embodiment also encompasses proteins encoded by polynucleotides.

Further provided is a vector, in particular an expression vector, comprising the isolated polynucleotide or nucleic acid molecule for the ferritin binding protein or the fusion protein comprising the ferritin binding protein as described herein, as well as a host cell comprising the isolated polynucleotide or the expression vector. For example, one or more polynucleotides, which encode the ferritin binding protein or the fusion protein comprising the ferritin binding protein as disclosed herein may be expressed in a suitable host and the produced protein can be isolated. A vector means any molecule or entity (*e.g*., nucleic acid, plasmid, bacteriophage or virus) that can be used for transfer of protein-encoding information into a host cell. Suitable vectors that may be applied are known in the art.

Suitable host cells include prokaryotes or eukaryotes, for example a bacterial host cell, a yeast host cell or a non-human host cell carrying a vector. A suitable host may be a microbial expression host that is capable to express the protein of the invention. Suitable bacterial expression host cells or systems are known in the art. Various mammalian or insect cell culture systems as known in the art can also be employed to express recombinant proteins.

Furthermore, an isolated cell comprising a polynucleotide or nucleic acid, or a vector as disclosed herein is provided.

**Method of producing a protein of the invention.** In a further embodiment, a method for the production of the binding protein for ferritin or the fusion protein comprising the ferritin binding protein as described is provided, the method comprising the step(s): (i) culturing a (suitable) host cell under conditions suitable for the expression of the binding protein for ferritin or the fusion protein comprising the ferritin binding protein so as to obtain said binding protein for ferritin or said fusion protein comprising the ferritin binding protein; and (ii) optionally isolating said binding protein for ferritin or said fusion protein comprising the ferritin binding protein. Suitable conditions for culturing a prokaryotic or eukaryotic host are well known to a person skilled in the art.

The binding protein for ferritin or the fusion protein comprising the ferritin binding protein may be prepared by any conventional and well-known techniques such as plain organic synthetic strategies, solid phase-assisted synthesis techniques, or by commercially available automated synthesizers. They may also be prepared by conventional recombinant techniques, alone or in combination with conventional synthetic techniques.

In one embodiment, a method for the preparation of the binding protein for ferritin or the fusion protein comprising the ferritin binding protein is provided, as detailed above, said method comprising the steps: (i) providing a nucleic acid molecule encoding the ferritin binding protein or encoding the fusion protein comprising the ferritin binding protein; (ii) introducing said nucleic acid molecule into an expression vector; (iii) introducing said expression vector into a host cell; (iv) culturing the host cell in a culture medium; (v) subjecting the host cell to culturing conditions suitable for expression thereby producing the ferritin binding protein or the fusion protein comprising the ferritin binding protein; optionally (vi) isolating the polypeptide produced in step (v); and (vii) optionally conjugating the ferritin binding protein or the fusion protein comprising the ferritin binding protein to a solid matrix as described above. In various embodiments of the present invention the production of the binding protein for ferritin or the fusion protein comprising the ferritin binding protein is performed by cell-free *in vitro* transcription and translation.

The following Examples are provided for further illustration of the invention. The invention, however, is not limited thereto, and the following Examples merely show the practicability of the invention on the basis of the above description.

### EXAMPLES

The following Examples are provided for further illustration of the invention. The invention, however, is not limited thereto, and the following Examples merely show the practicability of the invention on the basis of the above description.

### EXAMPLE 1. Selection and screening

*Libraries.* Proprietary cDNA libraries based on stable Protein A-like variants (artifical mosaic proteins composed of fragments of Protein A domains and additional mutations) were synthesized in house by randomized oligonucleotides generated by synthetic trinucleotide phosphoramidites (ELLA Biotech) or synthesized extern by Geneart to achieve a well-balanced amino acid distribution with simultaneously exclusion of cysteine and other amino acid residues at randomized positions. For the following selection process by phage display, the corresponding cDNA library was amplified by PCR and ligated into a pCD33-OmpA phagemid. Aliquots of the ligation mixture were used for electroporation of *E. coli* SS320 (Lucigen) to produce and purify the phage library to store them as cryo-stocks.

*Selection by phage display:* Unless otherwise indicated, established recombinant genetic methods were used. Naive libraries were enriched against human ferritin (ordered by Calbiochem, CAS 9007-73-2) as ON-target using phage display as selection system. In each round a preselection step was performed using empty Sigmablocker-blocked magnetic Dynabeads^{™} M-270 Epoxy. The AIT-method was applied, which means that the ON-target protein was immobilized to magnetic Dynabeads^{™} M-270 Epoxy before each round started. *E. coli* SS320 (Lucigene) were used for infection with cryo phage libraries and *E. coli* ER2738 (Lucigene) for reamplification of phage pools after each round. Amplification and purification of the phages were carried out using standard methods known to a skilled person. All three selection rounds were performed with the automated KingFisher-System (Thermo Fisher) to isolate and capture the desired phage-target complexes. Target concentration started at 40 nM (round 1) and declined each round down to 10 nM (round 3). Bound phages were eluted by trypsin and reamplified. The success of the selection was analyzed by phage-pool-ELISA in medium binding microtiter plate (Greiner Bio-One) coated with ferritin (125 ng/well), hIgG₁-Fc (125 ng/well), BSA (125 ng/well) or Sigmablocker. Bound phages were detected using α-M13 HRP conjugated antibody (GE Healthcare).

*Cloning of target binding phage pools into an expression vector.* Selection pools showing specific binding to ferritin in phage pool ELISA were amplified by PCR according to methods known in the art, cut with appropriate restriction nucleases and ligated into a derivative of the expression vector pET-28a (Merck, Germany) comprising an N-terminal GFP-10xHis-tag followed by a TVMV cleavage site and a C-terminal cysteine.

*Results:* Various phage display selection pools resulted in specific signals for the respective ON-target ferritin. Controls with BSA or Sigmablocker showed for almost all pools no binding. Selected pools were sequenced and subcloned for high throughput screening.

*Primary screening*: 8 Selection pools were proceeded to high-throughput screening vs. ferritin. Therefore ferritin [c= 3.0 µg/ml] was immobilized on a 384-well high-binding plate and bound variants were detected by fluorescence signal (ex 485 nm/em 535 nm). 2141 variants were selected for secondary screening vs ferritin (ON-target) and BSA (OFF-target) whereby the binding of variants was detected by fluorescence signal (ex 485 nm/em 535 nm). The specific variants were selected for sequencing, µ-scale purification and BLI analysis. Hit criteria: soluble protein expression, signal of sample >5.000 and 5-fold larger than signal of OFF-target.

*µ-scale purification and BLI analysis:* 376 variants arrived from secondary screening were defined as hit for µ-scale purification and BLI analysis vs ferritin using an Octet 8 channel system. For BLI measurements variants were immobilized via His-Tag on the surface of Ni-NTA sensors (ForteBio). Upon binding, variants were accumulated on the surface increasing the refractive index. This change in the refractive index was measured in real time and plotted as nm shift versus time. Affinity of captured variants vs ferritin [c= 200 nM] was determined and also plotted as nm shift versus time. These proteins were selected for subcloning in a suitable format (fusion protein) for affinity chromatography applications and for production in larger scale.

### EXAMPLE 2. Expression and purification of fusion proteins comprising ferritin binding proteins

Fusion proteins comprising ferritin binding proteins were expressed in an *Escherichia coli* BL21(DE3) fermentation process using a pNP-016 vector system under regulation of a T7 promoter. Seed cultures were grown in preculture medium (34.5 g/L yeast extract, 0.61 g/L MgSO₄, 14.2 K₂HPO₄, 0.5 g/L NH₄Cl, 50 µg/mL kanamycin). The fermentation process was performed in parallel bench-top bioreactors as a fed-batch process. The culture medium (17.25 g/L yeast extract, 0.61 g/L MgSO₄, 14.2 K₂HPO₄, 0.5 g/L NH₄Cl, 50 µg/mL kanamycin) was inoculated with seed culture and the culture grown until the substrate was depleted (37 °C, pH 7.1, 30 % pO2 saturation, aeration 2 VVM). Exponential feeding was performed with glucose as the main substrate (200 g/L glucose, 276 g/L yeast extract, 1.1 g/L MgSO₄, 50 µg/mL kanamycin). Protein expression was induced by isopropyl β-D-1-thiogalactopyranoside (IPTG, end concentration of 1 mM) at 30 °C for 5 h at a constant feeding rate. To collect biomass cells were centrifuged at 12,000 x g for 30 min. Bacterial pellets were stored at -20 °C before processing. Expression was analyzed via SDS-PAGE.

Fusion proteins 219124 and 219125 were purified by cation exchange chromatography and size exclusion. After cell disruption pH was adjusted to 3.0 and 2.5, respectively, to precipitate most of the host proteins. The initial capturing step was performed at pH 3.5 using SP Sepharose HP (Cytiva; binding buffer: 20 mM citric acid, 1 mM EDTA pH 3.5; elution buffer: 20 mM citric acid, 1 mM EDTA, 1 M NaCl pH 3.5) followed by size exclusion chromatography (Superdex 75 26/600, Cytiva) in 20 mM citric acid, 150 mM NaCl, 1 mM EDTA pH 6.0 carried out on an ÄKTA avant system (Cytiva).

Fusion proteins 219126 and 219127 were purified by gel filtration. After cell disruption acetic acid was added to a final concentration of 100 mM followed by a pH-adjustment to pH 2.5 and 2.0, respectively. The final polishing step was performed by size exclusion chromatography (Sephacryl 200HR XK26/70, Cytiva) in 20 mM citric acid, 150 mM NaCl, 1 mM EDTA pH 6.0 using an ÄKTA avant system (Cytiva).

Following SDS-PAGE analysis positive fractions were pooled and protein concentrations were determined by absorbance measurement at 280 nm using the molar absorbent coefficient. Further analysis included RP-HPLC and SE-HPLC. Reversed phase chromatography (RP-HPLC) has been performed using an Ultimate 3000 HPLC system (Thermo Fisher Scientific) and a PLRP-S (5 µm, 300 Å) column (Agilent). The resulting purity of all proteins was 100 %. Analytic size exclusion chromatography (SE-HPLC) has been performed using an Ultimate 3000 HPLC system (Thermo Fisher Scientific) and a Superdex75 increase 5/150 GL (Cytiva). No aggregation or oligomers were obtained. Final yields for the proteins were 0.95-4.14 mg per g wet biomass.

### EXAMPLE 3. Binding analysis of fusion proteins by SPR

The purified fusion proteins were immobilized on a sensor chip CM5 (Cytiva) or High Capacity Amine sensor chip (Bruker) using PDEA after NHS/EDC activation with Biacore 3000 SPR or Sierra SPR-32 system (Bruker). The chip was equilibrated with SPR running buffer (PBS 0.05 % Tween pH 7.3 or 10 mM sodium acetate, 150 mM NaCl, 0.05 % Tween, pH 4.5). Upon binding, target analyte was accumulated on the surface increasing the refractive index. This change in the refractive index was measured in real time and plotted as response or resonance units versus time. The analyte ferritin protein was applied to the chip in serial dilutions with a flow rate of 30 µl/min. The association was performed for 120 seconds and the dissociation for 120 seconds. After each run, the chip surface was regenerated with 30 µl regeneration buffer (6 M guanidine-hydrochloride pH 4.0) and equilibrated with running buffer. Binding studies were carried out by the use of Biacore 3000 Control Software or the Sierra SPR-32 system (Bruker); data evaluation was operated via the BIAevaluation or Sierra Analyser software, provided by the manufacturer, by the use of the Langmuir 1:1 model (RI=0). Evaluated dissociation constants (K_{D}) were standardized against the immobilized protein and indicated. Shown is the change in refractive index measured in real time and plotted as response or resonance unit [RU] versus time [sec]. Fusion proteins bind to ferritin with a K_{D} below 1 nM at pH 4.5 and at pH 7.3 (see **Table 1).** **FIGURE 2** shows the high affinity binding of fusion protein 219127 to ferritin at pH 4.5.

**Table 1. Binding affinity of fusion proteins comprising ferritin binding proteins**

| SEQ ID NO: | CID | comprises ferritin binding protein of SEQ ID NO: | K_{D} for ferritin (in nM) pH 7.3 | K_{D} for ferritin (in nM) pH 4.5 |
|---|---|---|---|---|
| 5 | 219126 | 2 | < 0.5 | < 0.5 |
| 6 | 219127 | 2 (dimer) | < 0.5 | < 0.5 |
| 7 | 219124 | 3 | < 0.5 | < 0.5 |
| 8 | 219125 | 3 (dimer) | < 0.5 | 0.9 |

### EXAMPLE 4. Affinity purification of ferritin

**Coupling parameter.** Fusion proteins 219124, 219126 and 219127 were purified to homogeneity and immobilized at 20 mg per mL activated Praesto^{™} Epoxy 85 (Purolite) according to the manufacturer's instructions, coupling conditions: 35 °C for 3 h, pH 9.5 per mL resin. All ligands were successfully coupled to epoxy-activated Praesto 85 resin. The coupling density was between 9.9 and 16.3 mg/ml for coupled ligands.

**Affinity chromatography with ferritin.** For affinity chromatography, Praesto85-219127 was packed into superformance column housing (Götec, 5-50) and equilibrated in 1xPBS pH 7.3. Resin was loaded with 30 mL of a solution containing ferritin (e.g. at least 500 µg human ferritin, Merck, catalog number: 341482) with residence times of 6 minutes. A wash step with 100 mM sodium acetate pH 4.5 was included to confirm that ferritin binds ligand also at pH 4.5. Elution was performed with a gradient from pH 4.5 to pH 2.0 in 15 column volumes (CV) using 100 mM citric acid. The pH of buffer fractions containing the target was determined.

Fusion protein 219127 (SEQ ID NO: 6, comprising SEQ ID NO: 2) showed a homogenous elution profile with a singular peak (see **FIGURE 3A****).**

The peak maximum was at pH 3.1 in a pH gradient profile. Eluted ferritin showed high purity on SDS-PAGE **(****FIGURE 3B****).** A strong enrichment of ferritin compared to the original load was observed. The identity of ferritin in elution fractions was confirmed by a specific ferritin ELISA (IBL international; catalog number: DB59111, according to manufacturer's inctructions)

**Static binding capacity (SBC).** The resin with immobilized fusion proteins 219124, 219126 or 219127 (Praesto 85_219214, Praesto85_219126, Praesto85_219127) was equilibrated in 1xPBS pH 7.3. Praesto 85_219214, Praesto85_219126, or Praesto85_219127 purified human ferritin (Merck, Catalog number: 341482; 1.5 mg/ml, 400 µl, in PBS pH 7.3) was mixed with 20 µl of the resin for 1 h at RT. The matrix was washed 2 times with 300 µl with 1x PBS pH 7.3 and the bound protein was eluted with 3 x 100 µl with 100 mM citric acid pH 2.0. The amount of eluted ferritin was calculated with ferritin ELISA (IBL international; catalog number: DB59111, according to manufacturer's inctructions).

The SBC was determined by the mass of eluted protein calculated. The static binding capacity was 5.6 mg/ml for both Praesto85_219126 and Praesto85_219127, respectively, and 9.6 mg/ml for Praesto85_219124.

### SEQUENCES

SEQ ID NO: 1: ferritin binding protein
   AKFDXXQXYADXXILXLXNLTEEQRNAFRQSLSDDPSVSWXVLXEARXLNEXQAPK
   X can be any naturally occurring amino acid. As used herein, X can be any one of A, R, N, D, C, Q, E, G, H, I, L, K, M, F, P, S, T, W, Y, or V.
SEQ ID NO: 2: ferritin binding protein (218990)
   AKFDQWQSYADHQILKLPNLTEEQRNAFRQSLSDDPSVSWEVLWEARELNESQAPK
SEQ ID NO: 3: ferritin binding protein (218989)
   AKFDAYQSYADKEILQLPNLTEEQRNAFRQSLSDDPSVSWEVLQEARVLNESQAPK
SEQ ID NO: 4: ferritin binding protein (218894)
   AKFDQFQLYADKEILELFNLTEEQRNAFRQSLSDDPSVSWYVLWEARMLNEAQAPK
SEQ ID NO: 5: fusion protein comprising SEQ ID NO: 2 (219126) 
AKFDQWQSYADHQILKLPNLTEEQRNAFRQSLSDDPSVSWEVLWEARELNESQAPKIAAQHD
KIQQAADKEILHLPNLTEEQRNKFRQSLRDDPSVSAEILAEAKKLNDAQAPKIAAQHDKIQQAAD
KEILHLPNLTEEQRNKFRQSLRDDPSVSAEILAEAKKLNDAQAPKC
SEQ ID NO: 6: fusion protein comprising SEQ ID NO: 2 (dimer)(219127) 
AKFDQWQSYADHQILKLPNLTEEQRNAFRQSLSDDPSVSWEVLWEARELNESQAPKIAAKFD
QWQSYADHQILKLPNLTEEQRNAFRQSLSDDPSVSWEVLWEARELNESQAPKIAAQHDKIQQ
AADKEILHLPNLTEEQRNKFRQSLRDDPSVSAEILAEAKKLNDAQAPKIAAQHDKIQQAADKEIL
HLPNLTEEQRNKFRQSLRDDPSVSAEILAEAKKLNDAQAPKC
SEQ ID NO: 7: fusion protein comprising SEQ ID NO: 3 (219124) 
AKFDAYQSYADKEILQLPNLTEEQRNAFRQSLSDDPSVSWEVLQEARVLNESQAPKIAAQHDKI
QQAADKEILHLPNLTEEQRNKFRQSLRDDPSVSAEILAEAKKLNDAQAPKIAAQHDKIQQAADK
EILHLPNLTEEQRNKFRQSLRDDPSVSAEILAEAKKLNDAQAPKC
SEQ ID NO: 8: fusion protein comprising SEQ ID NO: 3 (dimer)(219125) 
AKFDAYQSYADKEILQLPNLTEEQRNAFRQSLSDDPSVSWEVLQEARVLNESQAPKIAAKFDA
YQSYADKEILQLPNLTEEQRNAFRQSLSDDPSVSWEVLQEARVLNESQAPKIAAQHDKIQQAA
DKEILHLPNLTEEQRNKFRQSLRDDPSVSAEILAEAKKLNDAQAPKIAAQHDKIQQAADKEILHL
PNLTEEQRNKFRQSLRDDPSVSAEILAEAKKLNDAQAPKC
SEQ ID NO: 9: fusion protein comprising SEQ ID NO: 4 (219122) 
AKFDQFQLYADKEILELFNLTEEQRNAFRQSLSDDPSVSWYVLWEARMLNEAQAPKIAAQHDKI
QQAADKEILHLPNLTEEQRNKFRQSLRDDPSVSAEILAEAKKLNDAQAPKIAAQHDKIQQAADK
EILHLPNLTEEQRNKFRQSLRDDPSVSAEILAEAKKLNDAQAPKC
SEQ ID NO: 10: fusion protein comprising SEQ ID NO: 4 (dimer)(219123) 
AKFDQFQLYADKEILELFNLTEEQRNAFRQSLSDDPSVSWYVLWEARMLNEAQAPKIAAKFDQ
FQLYADKEILELFNLTEEQRNAFRQSLSDDPSVSWYVLWEARMLNEAQAPKIAAQHDKIQQAA
DKEILHLPNLTEEQRNKFRQSLRDDPSVSAEILAEAKKLNDAQAPKIAAQHDKIQQAADKEILHL
PNLTEEQRNKFRQSLRDDPSVSAEILAEAKKLNDAQAPKC
SEQ ID NO: 11: non ferritin binding protein
   AQHDKIQQAADKEILHLPNLTEEQRNKFRQSLRDDPSVSAEILAEAKKLNDAQAPK
SEQ ID NO: 12: ferritin

## Claims

1. A binding protein for ferritin comprising an amino acid sequence with at least 90 % sequence identity to SEQ ID NO: 2 or SEQ ID NO: 3, wherein the binding protein has a binding affinity of less than 100 nM for ferritin.

2. The binding protein of claim 1, wherein 2, 3, 4, 5, or 6 binding proteins are linked to each other.

3. The binding protein of claims 1-2 wherein the binding protein is fused to at least one non-ferritin binding protein.

4. A fusion protein comprising at least one binding protein according to claim 1 and at least one non-ferritin binding protein.

5. The fusion protein according to claim 4 wherein said fusion protein comprises an attachment site for site-specific coupling to a solid support.

6. The binding protein for ferritin according to any one of claims 1-3 or the fusion protein of claims 4-5 for use in technical applications such as affinity chromatography.

7. An affinity separation matrix comprising a binding protein for ferritin according to any one of claims 1-3 or the fusion protein of claims 4-5.

8. Use of the binding protein for ferritin according to any one of claims 1-3, or the fusion protein of claims 4-5 or the affinity separation matrix according to claim 7 for affinity chromatography.

9. A method of purification of ferritin, the method comprising:
(i) providing a liquid that contains a ferritin;
(ii) providing an affinity separation matrix comprising at least one binding protein for ferritin according to any one of claims 1-3 or the fusion protein of claims 4-5 coupled to said affinity separation matrix;
(iii) contacting said affinity separation matrix with the liquid under conditions that permit binding of the at least one binding protein for ferritin according to any one of claims 1-3 or the fusion protein of claims 4-5; and
(iv) eluting said ferritin from said affinity purification matrix.

10. Use of the binding protein for ferritin according to claims 1-3 or the fusion protein of claims 4-5 or the affinity matrix of claim 7 in methods to analyze the presence of ferritin in a liquid sample.

11. A method of analyzing the presence of ferritin in liquid samples, the method comprising the following steps:
(i) providing a liquid that contains ferritin,
(ii) providing the binding protein for ferritin according to claims 1-3 or the fusion protein of claims 4-5,
(iii) contacting the liquid of (i) with the binding protein for ferritin according to claims 1-3 or the fusion protein of claims 4-5 under conditions that permit binding of the binding protein to the ferritin,
(iv) isolating the complex of ferritin and binding protein, and
(v) determining the amount of the binding protein for ferritin in the liquid of (i).

12. A nucleic acid molecule encoding the binding protein for ferritin of claims 1-3 or the fusion protein of claims 4-5.

13. A vector comprising the nucleic molecule of claim 12.

14. A host cell or a non-human host comprising the binding protein for ferritin of claims 1-3 or the fusion protein of claims 4-5, a nucleic acid of claim 12, and/or a vector of claim 13.

15. A method of production of the binding protein for ferritin of claims 1-3 or the fusion protein of claims 4-5, comprising culturing of the host cell of claim 14 under suitable conditions in order to obtain said ferritin binding protein or fusion protein comprising the ferritin binding protein and optionally isolating said ferritin binding protein or fusion protein comprising the ferritin binding protein.

## Patentansprüche

1. Ein Bindungsprotein für Ferritin umfassend eine Aminosäuresequenz mit mindestens 90 % Sequenzidentität zu SEQ ID NO: 2 oder SEQ ID NO: 3, wobei das Bindungsprotein eine Bindungsaffinität von weniger als 100 nM für Ferritin aufweist.

2. Das Bindungsprotein nach Anspruch 1, wobei 2, 3, 4, 5 oder 6 Bindungsproteine miteinander verbunden sind.

3. Das Bindungsprotein nach den Ansprüchen 1-2, wobei das Bindungsprotein mit mindestens einem Nicht-Ferritin-bindenden Protein fusioniert ist.

4. Ein Fusionsprotein umfassend mindestens ein Bindungsprotein gemäß Anspruch 1 und mindestens ein Nicht-Ferritin-bindendes Protein.

5. Das Fusionsprotein gemäß Anspruch 4, wobei das Fusionsprotein eine Bindungsstelle für die ortsspezifische Kopplung an einen festen Träger umfasst.

6. Das Bindungsprotein für Ferritin gemäß einem der Ansprüche 1-3, oder das Fusionsprotein gemäß den Ansprüchen 4-5, zur Verwendung in technischen Anwendungen wie der Affinitätschromatographie.

7. Eine Affinitätstrennungsmatrix umfassend ein Bindungsprotein für Ferritin gemäß einem der Ansprüche 1-3 oder das Fusionsprotein gemäß den Ansprüchen 4-5.

8. Verwendung des Bindungsproteins für Ferritin gemäß einem der Ansprüche 1-3, oder des Fusionsproteins gemäß den Ansprüchen 4-5, oder der Affinitätstrennungsmatrix gemäß Anspruch 7, für die Affinitätschromatographie.

9. Verfahren zur Aufreinigung von Ferritin, wobei das Verfahren umfasst:
(i) Bereitstellen einer Flüssigkeit, welche Ferritin enthält;
(ii) Bereitstellen einer Affinitätstrennungsmatrix umfassend mindestens ein Bindungsprotein für Ferritin gemäß einem der Ansprüche 1-3 oder das Fusionsprotein gemäß den Ansprüchen 4-5, welches an die Affinitätstrennungsmatrix gekoppelt ist;
(iii) Inkontaktbringen der Affinitätstrennungsmatrix mit der Flüssigkeit unter Bedingungen, welche die Bindung des mindestens einen Bindungsproteins für Ferritin gemäß einem der Ansprüche 1-3 oder des Fusionsproteins gemäß den Ansprüchen 4-5 ermöglichen; und
(iv) Eluieren des Ferritins von der Affinitätsaufreinigungsmatrix.

10. Verwendung des Bindungsproteins für Ferritin gemäß den Ansprüchen 1-3, oder des Fusionsproteins gemäß den Ansprüchen 4-5, oder der Affinitätsmatrix gemäß Anspruch 7, in Verfahren zur Analyse des Vorhandenseins von Ferritin in einer flüssigen Probe.

11. Verfahren zur Analyse des Vorhandenseins von Ferritin in flüssigen Proben, wobei das Verfahren die folgenden Schritte umfasst:
(i) Bereitstellen einer Flüssigkeit, welche Ferritin enthält,
(ii) Bereitstellen des Bindungsproteins für Ferritin gemäß den Ansprüchen 1-3, oder des Fusionsproteins gemäß den Ansprüchen 4-5,
(iii) Inkontaktbringen der Flüssigkeit aus (i) mit dem Bindungsprotein für Ferritin gemäß den Ansprüchen 1-3, oder dem Fusionsprotein gemäß den Ansprüchen 4-5, unter Bedingungen, welche die Bindung des Bindungsproteins an das Ferritin ermöglichen,
(iv) Isolieren des Komplexes aus Ferritin und Bindungsprotein, und
(v) Bestimmen der Menge des Bindungsproteins für Ferritin in der Flüssigkeit aus (i).

12. Nukleinsäuremolekül, welches das Bindungsprotein für Ferritin gemäß den Ansprüchen 1-3 oder das Fusionsprotein gemäß den Ansprüchen 4-5 codiert.

13. Vektor, welcher das Nukleinsäuremolekül gemäß Anspruch 12 umfasst.

14. Wirtszelle oder ein nicht-menschlicher Wirt, umfassend das Bindungsprotein für Ferritin gemäß den Ansprüchen 1-3 oder das Fusionsprotein gemäß den Ansprüchen 4-5, eine Nukleinsäure gemäß Anspruch 12, und/oder einen Vektor gemäß Anspruch 13.

15. Verfahren zur Herstellung des Bindungsproteins für Ferritin gemäß den Ansprüchen 1-3, oder des Fusionsproteins gemäß den Ansprüchen 4-5, umfassend das Züchten der Wirtszelle nach Anspruch 14 unter Bedingungen, welche geeignet sind, das Ferritin-Bindungsprotein oder das Fusionsprotein, welches das Ferritin-Bindungsprotein umfasst, zu erhalten, und gegebenenfalls das Isolieren des Ferritin-Bindungsproteins oder des Fusionsproteins, welches das Ferritin-Bindungsprotein umfasst.

## Revendications

1. Protéine de liaison pour la ferritine comprenant une séquence d'acides aminés présentant au moins 90 % d'identité de séquence avec SEQ ID NO: 2 ou SEQ ID NO: 3, dans laquelle la protéine de liaison a une affinité de liaison inférieure à 100 nM pour la ferritine.

2. La protéine de liaison selon la revendication 1, dans laquelle 2, 3, 4, 5 ou 6 protéines de liaison sont liées les unes aux autres.

3. La protéine de liaison des revendications 1-2, dans laquelle la protéine de liaison est fusionnée à au moins une protéine de liaison non ferritine.

4. Protéine de fusion comprenant au moins une protéine de liaison selon la revendication 1 et au moins une protéine de liaison non ferritine.

5. La protéine de fusion selon la revendication 4, dans laquelle ladite protéine de fusion comprend un site de fixation pour un couplage spécifique à un support solide.

6. La protéine de liaison pour la ferritine selon l'une quelconque des revendications 1 à 3, ou la protéine de fusion selon les revendications 4 à 5, destinée à être utilisée dans des applications techniques telles que la chromatographie d'affinité.

7. Matrice de séparation par affinité comprenant une protéine de liaison pour la ferritine selon l'une quelconque des revendications 1 à 3, ou la protéine de fusion selon les revendications 4 à 5.

8. Utilisation de la protéine de liaison pour la ferritine selon l'une quelconque des revendications 1 à 3, ou de la protéine de fusion selon les revendications 4 à 5, ou de la matrice de séparation par affinité selon la revendication 7, pour la chromatographie d'affinité.

9. Procédé de purification de la ferritine, le procédé comprenant:
(i) la fourniture d'un liquide contenant une ferritine;
(ii) fournir une matrice de séparation par affinité comprenant au moins une protéine de liaison pour la ferritine selon l'une quelconque des revendications 1 à 3, ou la protéine de fusion selon les revendications 4 à 5, couplée à ladite matrice de séparation par affinité;
(iii) mettre en contact ladite matrice de séparation par affinité avec le liquide dans des conditions qui permettent la liaison de la au moins une protéine de liaison pour la ferritine selon l'une quelconque des revendications 1 à 3, ou la protéine de fusion selon les revendications 4 à 5; et
(iv) éluer ladite ferritine à partir de ladite matrice de purification par affinité.

10. Utilisation de la protéine de liaison pour la ferritine selon les revendications 1 à 3, ou de la protéine de fusion selon les revendications 4 à 5, ou de la matrice d'affinité selon la revendication 7, dans des méthodes d'analyse de la présence de ferritine dans un échantillon liquide.

11. Procédé d'analyse de la présence de ferritine dans des échantillons liquides, le procédé comprenant les étapes suivantes:
(i) fournir un liquide contenant de la ferritine,
(ii) fournir la protéine de liaison pour la ferritine selon les revendications 1 à 3, ou la protéine de fusion selon les revendications 4 à 5,
(iii) mettre en contact le liquide de (i) avec la protéine de liaison pour la ferritine selon les revendications 1 à 3, ou la protéine de fusion selon les revendications 4 à 5, dans des conditions qui permettent la liaison de la protéine de liaison à la ferritine,
(iv) isoler le complexe de ferritine et de protéine de liaison, et
(v) déterminer la quantité de protéine de liaison pour la ferritine dans le liquide de (i).

12. Molécule d'acide nucléique codant pour la protéine de liaison à la ferritine selon les revendications 1 à 3, ou la protéine de fusion selon les revendications 4 à 5.

13. Vecteur comprenant la molécule d'acide nucléique de la revendication 12.

14. Cellule hôte ou hôte non humain comprenant la protéine de liaison à la ferritine des revendications 1 à 3, ou la protéine de fusion des revendications 4 à 5, un acide nucléique de la revendication 12 et/ou un vecteur de la revendication 13.

15. Procédé de production de la protéine de liaison à la ferritine selon les revendications 1 à 3, ou de la protéine de fusion selon les revendications 4 à 5, comprenant la culture de la cellule hôte selon la revendication 14 dans des conditions appropriées afin d'obtenir ladite protéine de liaison à la ferritine ou ladite protéine de fusion comprenant la protéine de liaison à la ferritine et, éventuellement, l'isolement de ladite protéine de liaison à la ferritine ou de ladite protéine de fusion comprenant la protéine de liaison à la ferritine.
